# EUROPÄISCHE PATENTSCHRIFT

(11) **EP 0 863 731 B1**
(45) Veröffentlichungstag und Bekanntmachung des Hinweises auf die Patenterteilung: **18.04.2001**
(21) Anmeldenummer: 96938009.6
(22) Anmeldetag: 04.09.1996
(51) Int. Cl.: A61F 2/36

(54) **MODULARE ENDOPROTHESE**
MODULAR ENDOPROSTHESIS
ENDOPROTHESE MODULAIRE

(30) Priorität: 20.11.1995 DE 19544168
(43) Veröffentlichungstag der Anmeldung: 16.09.1998
(73) Patentinhaber: ARTOS Medizinische Produkte GmbH, 12277 Berlin (DE)
(72) Erfinder: KRANZ, Curt, D-10825 Berlin (DE)
(74) Vertreter: Maikowski, Michael, Dipl.-Ing. Dr.
(86) Internationale Anmeldenummer: DE9601713
(87) Internationale Veröffentlichungsnummer: WO9718776

(56) Entgegenhaltungen:
- EP-A- 0 332 571
- EP-A- 0 457 222
- EP-A- 0 550 117
- EP-A- 0 611 558
- WO-A-96/15739
- DE-A- 3 340 767
- DE-A- 3 802 213
- DE-A- 4 031 520
- DE-U- 8 903 850
- DE-U- 29 506 036
- FR-A- 2 606 628
- US-A- 4 808 186

## Beschreibung

Die Erfindung betrifft eine modulare Endoprothese, insbesondere eine Femurprothese, gemäß dem Oberbegriff des Anspruchs 1.

Aus DE 33 40 767 A1 ist eine derartige Prothese bekannt, welche aus mindestens zwei Schaftteilen besteht, die durch Zusammenstecken verbunden werden. Die mechanische Verbindung zwischen zwei Schaftelementen erfolgt hierbei dadurch, daß ein an das eine Schaftelement angeformter konischer Zapfen in eine in dem anderen Schaftelement angeordnete konische Bohrung eingepreßt wird und mit dieser eine Preßpassung bildet.

Wegen der Zusammensetzung der Endoprothese aus unterschiedlichen Schaftelementen lassen sich so vorteilhaft Endoprothesen unterschiedlicher Schaftlänge zusammensetzen.

Darüber hinaus ermöglicht die Endoprothese eine einfache Entfernung, beispielsweise im Falle einer Reoperation, indem die Schaftelemente voneinander getrennt und einzeln entfernt werden.

Nachteilig bei der vorbekannten Endoprothese ist jedoch, daß das die Konusaufnahme aufweisende Schaftelement im Bereich seines Mündungsrands durch Mikrobewegungen hervorgerufenen Verschleiß aufweisen kann, welcher im Extremfall zu Rissen führen kann.

Der Erfindung liegt deshalb die Aufgabe zugrunde, bei einer derartigen modularen Endoprothese diese Verschleißerscheinungen zu verhindern.

Die Aufgabe wird durch die kennzeichnenden Merkmale des Anspruchs 1 gelöst.

Die Erfindung schließt die technische Lehre ein, bei einer derartigen Endoprothese das Schaftelement mit der konischen Bohrung derart auszuformen, daß lokale Spitzen der mechanischen Spannung im Bereich des Mündungsrandes der Bohrung vermieden werden.

Die Erfindung geht dabei von der Erkenntnis aus, daß das bekannte, die Konusaufnahme aufweisende, Schaftelement im Randbereich zu starr ausgebildet war, was bei einer Biegebelastung der Endoprothese mit einer entsprechenden Verschränkung der Längsachsen von Zapfen und Bohrung zu einer Verringerung der wirksamen Reibkräfte zwischen Zapfen und Bohrung und damit zu lokalen Mikrobewegungen führte.

So entspricht zwar die spannungsaufnehmende Kontaktfläche bei Biegebelastung der Endoprothese im wesentlichen der gesamten Mantelfläche des konischen Zapfens. Mit ansteigender Biegebelastung und entsprechend zunehmender Verschränkung der Längsachsen von Zapfen und Bohrung reduzieren sich dann die übertragbaren Reibkräfte. Dies führt am Mündungsrand der Bohrung zu lokalen Bewegungen, welche zu Abrieb und zur Rißbildung führen können.

Da die auf die Endoprothese wirkende Biegebelastung zeitlich nicht konstant ist, sondern hinsichtlich Betrag und Richtung entsprechend den natürlichen Belastungszuständen der Endoprothese Schwankungen unterliegt, treten zwischen Zapfen und Bohrung oszillierende Mikrobewegungen auf. Diese Mikrobewegungen führen in Verbindung mit den am Mündungsrand der Bohrung auftretenden lokalen Spannungsspitzen zu einem Materialabtrag und damit zu einem frühzeitigen Verschleiß, was auch als Fretting bezeichnet wird.

Gemäß der Erfindung wird deshalb die Elastizität des Schaftelements kurz vor dem Mündungsende der Kegelbohrung erhöht, damit sich die Innenwand der Bohrung bei einer Biegebelastung der Endoprothese und damit zusammenhängend einer Verschränkung der Längsachsen von Zapfen und Bohrung an die Stellung des Zapfens anpaßt und mit der Mantelfläche des Zapfens eine hinreichend große umfangsspannungsaufnehmende Kontaktfläche bildet. Das unmittelbare Ende des Konus behält jedoch seine Wandungsstärke, um hier eine ausreichende Festigkeit in Umfangsrichtung beizubehalten. Somit wird erreicht, daß die Krafteinleitung im Endbereich des Konus über eine größere Länge verteilt wird und somit eine Überbeanspruchung im unmittelbaren Mündungsbereich vermieden ist.

Gemäß der Erfindung weist das mit der konischen Bohrung versehene Schaftelement deshalb an der Außenwandung im Bereich der Bohrung in der Nähe des Mündungsendes eine in Bezug auf die Längsachse der Bohrung umlaufende Einkerbung auf.

Diese Einkerbung bzw. die damit verbundene Verringerung der Wandstärke des Schaftelements im Bereich der Bohrung führt zu einer Erhöhung der Elastizität des Schaftelements, so daß sich die Innenwand der Bohrung bei einer Verschränkung des Zapfens relativ zur Bohrung an den Zapfen anpaßt.

Die Ausführung des Schaftelements mit einer sich über den gesamten Umfang erstreckenden Einkerbung ist vorteilhaft, da die erfindungsgemäße verschleißmindernde Wirkung dann unabhängig von der Richtung der Biegebelastung ist.

In einer vorteilhaften weiterbildenden Ausführungsform der Erfindung weist die Einkerbung einen glatten Verlauf mit hinreichend großen Krümmungsradien unter Vermeidung einspringender Ecken auf. Hierdurch wird die mechanische Spannung in der Außenwand des Schaftelements in der Einkerbung zusätzlich herabgesetzt und einer Rißbildung entgegengewirkt.

Die Einkerbung wird vorzugsweise derart ausgeformt, daß bei einer Biegebelastung des Schaftelements die im Bereich der Bohrung maximal auftretende mechanische Spannung möglichst vergleichmäßigt ist. Zur Bestimmung der optimalen Form der Einkerbung kann die in dem Schaftelement bei einer Biegebelastung auftretende mechanische Spannung beispielsweise mittels numerischer Simulationsverfahren für verschiedene Einkerbungsformen berechnet werden, von denen dann die die geringste Maximalspannung aufweisende Form ausgewählt wird.

In einer vorteilhaften Ausführungsform der Erfindung weist die Einkerbung eine entlang der Symmetrieachse zum Ende des Schaftelements hin zunehmende Tiefe auf. Dies bedeutet entsprechend, daß die Wandstärke des Schaftelements im Bereich der Bohrung zu seinem Ende hin abnimmt, während die Nachgiebigkeit der Zapfenaufnahme zum Ende des Schaftelements hin zunimmt. Dies ist günstig, weil die Zapfenaufnahme auf diese Weise bei einer nur geringen Verschränkung des Zapfens gegenüber der Bohrung sehr nachgiebig ist, was zu einer Abschwächung der lokalen Spannungsspitzen führt, andererseits aber bei zunehmender Verschränkung des Zapfens gegenüber der Bohrung härter wird und damit eine sichere Führung des Zapfens auch bei größeren Biegebelastungen sicherstellt.

Zur Erreichung einer guten Biokompatibiltät der Endoprothese ist eine glatte Oberfläche der zu implantierenden Teile ohne hervorspringende Kanten oder Ecken wünschenswert. Die Einkerbung in den Schaftelementen geht deshalb vorzugsweise glatt in die Außenwand über, ohne an der Übergangsstelle einen Knick oder gar eine Stufe zu bilden.

Die mechanische Verbindung zwischen zwei Schaftelementen erfolgt in der Regel kraftschlüssig, indem der Zapfen in dem einen Schaftelement und die Bohrung in dem anderen Schaftelement eine Preßpassung bilden.

Es kann jedoch wünschenswert sein, eine größere mechanische Festigkeit der Verbindung der Schaftelemente zu erreichen.

In einer weiterbildenden Variante der Erfindung weisen die Schaftelemente deshalb einen vorzugsweise mittig angeordneten Längskanal zur Aufnahme eines Zugankers auf, der eine Verspannung der einzelnen Schaftelemente gegeneinander ermöglicht.

Bei einer mittigen Anordnung des Längskanals wird die Biegebelastbarkeit der Endoprothese durch den Längskanal nur unwesentlich herabgesetzt, da bei einer Biegebelastung die Spannungsmaxima in den Schaftelementen randnah auftreten.

In einer bevorzugten Ausführungsform der Erfindung weist die Endoprothese deshalb eine Vielzahl von Schaftelementen auf, die zu einer Endoprothese zusammengesteckt werden können, so daß die Länge der Endoprothese um ein ganzzahliges Vielfaches der Länge eines Schaftelementes veränderbar ist.

Oftmals ist es jedoch erwünscht, die Länge der Endoprothese feiner abstufen zu können. In einer vorteilhaften weiterbildenden Variante der Erfindung weist die Endoprothese deshalb eine Vielzahl von Schaftelementen unterschiedlicher Länge auf. Dies ermöglicht zum einen die Zusammensetzung einer Endprothese mit einer vorgegebenen Länge aus sehr wenigen Schaftelementen und zum anderen eine sehr feine Abstufung der Endoprothesenlänge.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: als bevorzugtes Ausführungsbeispiel der Erfindung eine Endoprothese mit mehreren Schaftelementen im Querschnitt,
- Figur 2: ein Schaftelement der in Figur 1 dargestellten Endoprothese detailliert im Querschnitt sowie den Verlauf der in dem Schaftelement auftretenden mechanischen Spannung sowie
- Figur 3: die Einkerbung in dem Schaftelement aus Figur 2 detailliert im Querschnitt.

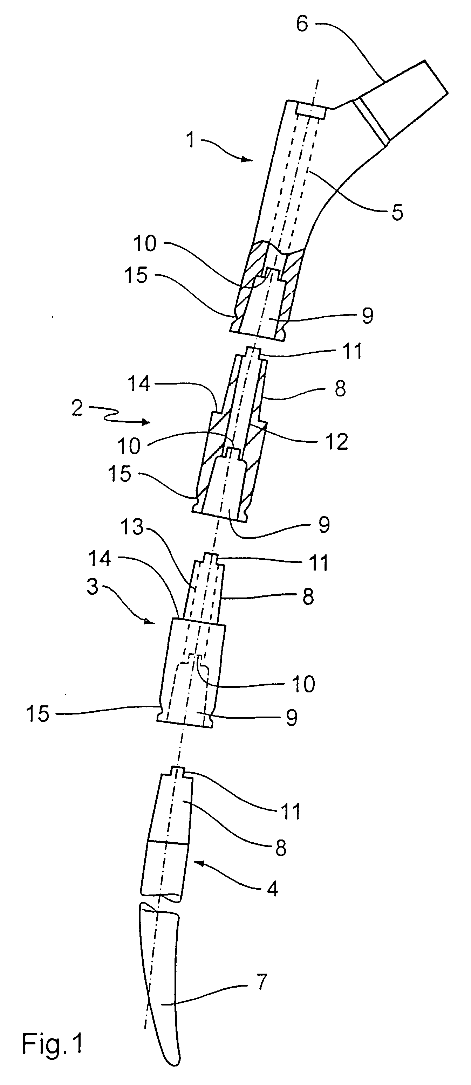

Die in Figur 1 dargestellte Endoprothese wird in den Femur eingefügt.

Die Endoprothese besteht im wesentlichen aus vier Schaftelementen 1, 2, 3, 4 von im wesentlichen kreisförmigem Querschnitt, die ineinandergesteckt werden und im zusammengesteckten Zustand eine feste Einheit bilden.

Das Schaftelement 1 - im folgenden als Kopfteil bezeichnet - weist an seiner Oberseite einen Zapfen 6 zur Aufnahme einer Gelenkkugel auf, die eine Lagerung in der Gelenkpfanne des Beckens ermöglicht.

Zur Erreichung einer optimalen Funktion der Endoprothese ist das Kopfteil 1 im oberen Bereich entsprechend der Form eines natürlichen Femur gekrümmt.

Ein anderes Schaftelement 4 - im folgenden als Endteil bezeichnet - dient zur Verankerung der Endoprothese in dem verbliebenen natürlichen Knochen. An der Unterseite des Endteils 4 ist deshalb ein sich zu seinem Ende hin verjüngender Schaft 7 angeformt, der dazu bestimmt ist, in den Markraum des verbliebenen natürlichen Knochens einzementiert zu werden.

Zur Verbindung untereinander weisen die Schaftelemente 2, 3 jeweils einen an der Oberseite angeformten konischen Zapfen 8 sowie eine an der Unterseite angeordnete konische Bohrung 9 auf, die an den Zapfen 8 angepaßt ist und mit diesem eine Preßpassung bildet. Beim Zusammenbau der Endoprothese wird also jeweils der Zapfen 8 eines Schaftelements in die Bohrung 9 eines anderen Schaftelements hineingepreßt.

Das Endteil 4 weist lediglich einen an der Oberseite angeformten Zapfen 8 auf, der in die Bohrung 9 des Schaftelements 3 eingepreßt wird. Entsprechend verfügt das Kopfteil 1 nur über eine an der Unterseite angeordnete Bohrung 9 zur Aufnahme des Zapfens 8 des oberen Schaftelements 2.

Die einzelnen Schaftelemente 2, 3, 4 sind wie das Kopfteil 1 entsprechend der natürlichen Form eines Femur leicht gekrümmt. Damit die Endoprothese der natürlichen Form eines Femur entspricht, müssen die einzelnen Schaftelemente 1, 2, 3, 4 deshalb zum einen in der richtigen Reihenfolge und zum anderen in der richtigen Winkelstellung zueinander ineinandergesteckt werden.

Um beim Zusammenbau der Endoprothese die richtige Winkelstellung sicherzustellen und eine Verdrehung der einzelnen Schaftelemente 1, 2, 3, 4 im implantierten Zustand zu verhindern, ist am Boden der konischen Bohrung 9 jeweils eine Nut 10 angeformt, in die eine an den Zapfen 8 angeformte Feder 11 beim Zusammenstecken zweier Schaftelemente eingreift und so eine Drehung verhindert.

Gelegentlich besteht die Notwendigkeit, eine bereits implantierte Endoprothese wieder zu entfernen, beispielsweise weil durch Verschleiß die Lebensdauer der Prothese ausgeschöpft ist oder weil der Patient durch zwischenzeitliches Körperwachstum eine größere Endoprothese benötigt. In einem derartigen Fall ist es wünschenswert, die Schaftelemente 1, 2, 3, 4 einzeln entfernen zu können, was die Handhabung bei der Entfernung der Endoprothese wesentlich erleichtert.

Hierzu müssen die Schaftelemente 1, 2, 3, 4 voneinander getrennt werden, was wegen der Preßpassung von Zapfen 8 und Bohrung 9 normalerweise einen erheblichen Kraftaufwand erfordert. Die Schaftelemente 1, 2, 3 weisen deshalb jeweils eine entlang der Längsachse durchlaufende Gewindebohrung 5, 12, 13 auf. Zur Trennung der Schaftelemente wird dann jeweils eine Schraube von oben in die Gewindebohrung hineingedreht, bis die Schraube den Zapfen 8 aus der Bohrung 9 herausdrückt.

Im implantierten Zustand werden die aufgrund der mechanischen Belastung der Endoprothese entstehenden Längskräfte von den aneinanderstoßenden Flächen 14 der Schaftelemente 2, 3 sowie von den Mantelflächen der konischen Zapfen 8 bzw. Bohrungen 9 aufgenommen. Darüber hinaus wirkt auf die Endoprothese bzw. die einzelnen Schaftelemente 1, 2, 3, 4 jedoch eine Biegebelastung, die insbesondere an den Kontaktflächen von Zapfen 8 und Bohrung 9 zu erhöhtem Verschleiß führt. Dies rührt daher, daß bei einer Biegebelastung der Endoprothese der normalerweise mit der Bohrung 9 fluchtende Zapfen 8 gegenüber der Bohrung 9 verschränkt wird, was zu einer Abnahme der wirksamen spannungsaufnehmenden Kontaktfläche zwischen Zapfen 8 und Bohrung 9 und damit zu lokalen Spannungsspitzen insbesondere am Mündungsrand der Bohrung 9 führt.

Die Schaftelemente 1, 2, 3 weisen deshalb an der Außenwand in Höhe der Bohrung 9 eine in Bezug auf die Längsachse umlaufende Einkerbung 15 auf, die die Elastizität der Zapfenaufnahme erhöht. Dies bedeutet, daß die Schaftelemente 1, 2, 3 bei einer auf die Endoprothese wirkenden Biegebelastung an der Mündung der Bohrung 9 dem Zapfen 8 geringfügig nachgeben, was zu einer Vergrößerung der wirksamen spannungsaufnehmenden Kontaktfläche und damit zu einer Verringerung der maximal auftretenden mechanischen Spannung führt.

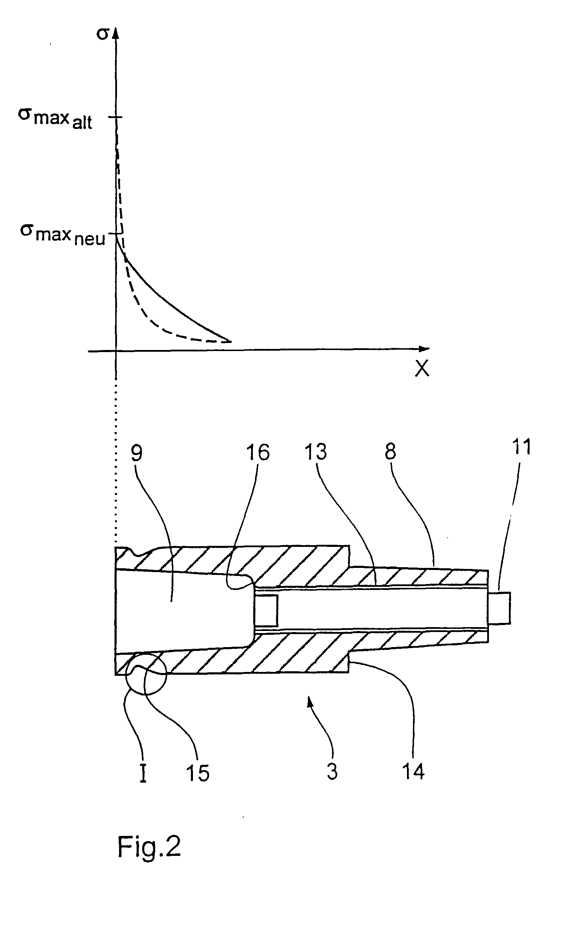

Figur 2 zeigt in ihrem unteren Teil das Schaftelement 3 der in Figur 1 dargestellten Endoprothese vergrößert mit der in der Außenwand angeordneten Einkerbung 15.

Deutlich zu sehen ist hier, daß am Boden der konischen Bohrung 9 ein Radius 16 angeformt ist, um die mechanischen Spannungen am Übergang von der Mantelfläche der konischen Bohrung 9 zum Boden der Bohrung 9 herabzusetzen.

Durch die Schwächung der Wandung infolge der Einkerbung erfolgt die Krafteinleitung nicht erst als maximale Spitze am Mündungsende, sondern bereits (vergleichmäßigt) vorher, vom Mündungsende im Bereich der Einkerbung. Durch die Verringerung der Spannungen wird eine Überlastung des Materials verhindert.

Die dargestellte Form der Einkerbung 15 stellt einen optimalen Kompromiß dar zwischen der Forderung nach einer großen Starrheit der Zapfenaufnahme zur Erreichung einer sicheren, weitgehend spielfreien Führung des Zapfens einerseits und andererseits der Forderung nach einer ausreichenden Nachgiebigkeit der Zapfenaufnahme, um lokale Spannungsspitzen am Mündungsrand der Bohrung 9 abzubauen und die Kräfte auf eine größere spannungsaufnehmende Kontaktfläche möglichst gleichmäßig zu verteilen.

Die Einkerbung 15 weist eine entlang der Längsachse der Bohrung 9 zum Ende des Schaftelements 3 hin zunehmende Tiefe auf. Entsprechend nimmt die Wandstärke des Schaftelements 3 im Bereich der Bohrung 9 zum Ende des Schaftelements 3 hin ab. Dies führt zu einer zum Ende des Schaftelements 3 hin zunehmenden Nachgiebigkeit.

Bei Biegebelastungen ist die Zapfenaufnahme deshalb relativ nachgiebig und paßt sich der veränderten Stellung des Zapfens gut an. Dies führt zu einer Vergrößerung des wirksamen spannungsaufnehmenden Kontaktflächenbereichs zwischen Zapfen und Bohrung 9 und damit zu einer Abschwächung lokaler Spannungsspitzen am Mündungsrand der Bohrung 9.

Weiterhin zeigt Figur 2 - in ihrem oberen Teil - in einem zugeordneten Diagramm den Verlauf der in der Zapfenaufnahme auftretenden mechanischen Spannung entlang der Längsachse der Bohrung (bezogen auf die Darstellung im unteren Teil von Figur 2). Die gestrichelte Linie zeigt hierbei zum Vergleich den Spannungsverlauf bei einer aus dem Stand der Technik vorbekannten Endoprothese, während die durchgezogene Linie den Verlauf der mechanischen Spannung bei der vorstehend beschriebenen erfindungsgemäßen Endoprothese zeigt.

Bei der vorbekannten Endoprothese nimmt die mechanische Spannung entlang der Längsachse der Bohrung sehr stark zu. So ist die Spannung im oberen Bereich der Bohrung relativ gering und nimmt in der Nähe des Mündungsrandes bis auf den Wert δ_{MAX,Alt} zu, was zu einer Überlastung führen kann.

Bei der erfindungsgemäßen Endoprothese ist der Spannungsverlauf entlang der Längsachse der Bohrung dagegen wesentlich gleichmäßiger, was sich vorteilhaft in einer wesentlich geringeren Maximalspannung δ_{Max,Neu} auswirkt. Dies wird dadurch erreicht, daß durch die mittels der Einkerbung verursachte Festigkeitsverringerung der Konushülse diese sich hier bei Belastung elastisch verformt, so daß eine Kraftüberleitung bereits bevorzugt im Bereich der Einkerbung erfolgen kann. Die entsprechenden Kräfte entfallen aber dafür am Konusende. Weil hier aber die ursprüngliche - nicht verringerte - Wandstärke vorhanden ist, ist dieser Bereich ausreichend stabil, um die verringerten Kräfte aufzunehmen.

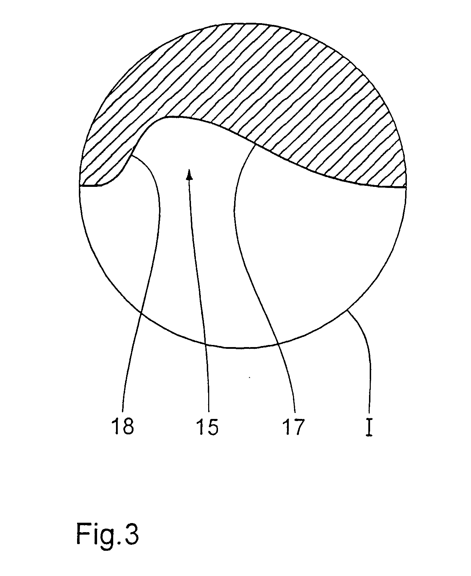

Die Form der Einkerbung 15 ist detailliert aus Figur 3 ersichtlich, die den Ausschnitt I aus Figur 2 wiedergibt. Diese Darstellung verdeutlicht, daß die Einkerbung 15 unsymmetrisch ist und eine zum Ende des Zapfens hin zunehmende Tiefe aufweist. Die Einkerbung 15 weist also zwei Flanken 17, 18 unterschiedlicher Steigung auf, wobei die dem Zapfenende zugewandte Flanke 18 relativ steil verläuft und nur eine geringe Längserstreckung aufweist, während die dem Zapfenende abgewandte Flanke 17 relativ flach, aber langgestreckt ist und in der Zapfenwand ausläuft.

## Patentansprüche

1. Modulare Endoprothese, insbesondere Femurprothese, welche eine Konus-Steckverbindung aufweist, mit
einem ersten Schaftelement (2) mit einer im wesentlichen konischen Bohrung (9),
einem zweiten Schaftelement (3) mit einem im wesentlichen konischen, an die Bohrung (9) in dem ersten Schaftelement (2) angepaßten Zapfen (8) zur Verbindung der beiden Schaftelemente (2, 3),
**dadurch gekennzeichnet,**
daß das die Bohrung aufweisende, erste Schaftelement (2) zur Verringerung der bei einer Biegebeanspruchung am Mündungsrand der Bohrung (9) auftretenden mechanischen Belastung an seiner Außenwand in der Nähe des Mündungsendes (9) eine umlaufende Einkerbung (15) aufweist.

2. Endoprothese nach Anspruch 1, **dadurch gekennzeichnet,** daß die Tiefe der Einkerbung (15) mindestens teilweise entlang der Längsachse der Bohrung (9) zum Ende des ersten Schaftelements (2) hin zunimmt.

3. Endoprothese nach einem der Ansprüche 1 oder 2, **dadurch gekennzeichnet,** daß die Einkerbung (15) derart geformt und angeordnet ist, daß die bei Biegebelastung auftretende mechanische Spannung am Mündungsrand der Bohrung (9) in Richtung der Längsachse der Bohrung (9) in Mündungsnähe im wesentlichen vergleichmäßigt ist.

4. Endoprothese nach einem der vorhergehenden Ansprüche, **dadurch gekennzeichnet,** daß die Schubspannungen vergleichmäßigt sind.

5. Endoprothese nach einem der vorhergehenden Ansprüche, **dadurch gekennzeichnet,** daß der Querschnitt der Einkerbung (15) derart ausgestaltet ist, daß deren Tiefe in Richtung auf die Mündung zu weniger steil zunimmt als sie anschließend wieder abnimmt.

6. Endoprothese nach einem der vorhergehende n Ansprüche, **dadurch gekennzeichnet,** daß mindestens ein Schaftelement (1, 2, 3, 4) ähnlich einem natürlichen Oberschenkelknochen gekrümmt ist.

7. Endoprothese nach einem der vorhergehenden Ansprüche, **dadurch gekennzeichnet,** daß Bohrung (9) und Zapfen (8) eine Preßpassung bilden.

8. Endoprothese nach einem der vorhergehenden Ansprüche, **dadurch gekennzeichnet,** daß die Schaftelemente (1, 2, 3, 4) jeweils einen Längskanal aufweisen zur Aufnahme eine Zugankers zur Verspannung der Schaftelemente.

9. Endoprothese nach einem der vorhergehenden Ansprüche, **dadurch gekennzeichnet,** daß ein Schaftelement (1) einen künstlichen Gelenkkopf (5) aufweist.

10. Endoprothese nach einem der vorhergehenden Ansprüche, **dadurch gekennzeichnet,** daß an ein Schaftelement (4) ein Knochennagel (7) angeformt ist zur Verankerung des Schaftelements (4) in einem natürlichen Knochen.

## Claims

1. Modular endoprosthesis, more particularly femur prosthesis, which has a cone and socket connection, with a first shaft element (2) having a substantially conical bore (9), and a second shaft element (3) which has a substantially conical stud (8) adapted to fit the bore (9) in the first shaft element (2) for the purpose of connecting the two shaft elements (2, 3) together,
characterised in that
the first shaft element (2) having the bore has on its outer wall close to the mouth end (9) a circumferential notch (15) in order to reduce the mechanical strain which occurs in the event of bending strain on the mouth edge of the bore (9).

2. Endoprosthesis according to claim 1 characterised in that the depth of the notch (15) increases at least in part along the longitudinal axis of the bore (9) towards the end of the first shaft element (2).

3. Endoprosthesis according to one of claims 1 or 2 characterised in that the notch (15) is shaped and arranged so that the mechanical strain on the mouth edge of the bore (9) which occurs in the event of bending strain in the area close to the mouth is substantially evened out in the direction of the longitudinal axis of the bore (9).

4. Endoprosthesis according to one of the preceding claims characterised in that the shear stresses are evened out.

5. Endoprosthesis according to one of the preceding claims characterised in that the cross-section of the notch (15) is shaped so that its depth in the direction towards the mouth increases less steeply than it then increases again.

6. Endoprosthesis according to one of the preceding claims characterised in that at least one shaft element (1, 2, 3, 4) is curved similar to a natural thigh bone.

7. Endoprosthesis according to one of the preceding claims characterised in that the bore (9) and stud (8) form a press fit.

8. Endoprosthesis according to one of the preceding claims characterised in that the shaft elements (1, 2, 3, 4) each have a longitudinal channel to hold a tie anchor for tensioning the shaft elements.

9. Endoprosthesis according to one of the preceding claims characterised in that a shaft element (1) has an artificial articulation head (5) .

10. Endoprosthesis according to one of the preceding claims characterised in that a bone nail (7) is formed on a shaft element (4) to anchor the shaft element (4) in the natural bone.

## Revendications

1. Endoprothèse modulaire, notamment prothèse de fémur, qui possède une liaison d'enfichage conique, comportant
un premier élément de tige (2) possédant un perçage essentiellement conique (9),
un second élément de tige (3) possédant un embout essentiellement conique (8) adapté au perçage (9) situé dans le premier élément de tige (2), pour relier les deux éléments de tige (2, 3),
caractérisée en ce
que le premier élément de tige (2) qui comporte le perçage, possède, une encoche périphérique (15) au niveau de sa paroi extérieure, à proximité de l'extrémité (9) de l'embouchure, de manière à réduire la contrainte mécanique, qui apparaît au niveau du bord de l'embouchure du perçage (9) dans le cas d'une contrainte de flexion.

2. Endoprothèse selon la revendication 1, caractérisée en ce que la profondeur de l'encoche (15) augmente au moins en partie le long de l'axe longitudinal du perçage (9) en direction de l'extrémité du premier élément de tige (2).

3. Endoprothèse selon l'une des revendications 1 ou 2, caractérisée en ce que l'encoche (15) est conformée et disposée de telle sorte que la contrainte mécanique, qui apparaît lors d'une charge en flexion, au niveau du bord de l'embouchure du perçage (9) dans la direction de l'axe longitudinal du perçage (9) est essentiellement uniformisée à proximité de l'embouchure.

4. Endoprothèse selon l'une des revendications précédentes, caractérisée en ce que les contraintes de poussée sont uniformisées.

5. Endoprothèse selon l'une des revendications précédentes, caractérisée en ce que la section transversale de l'encoche (15) est agencée de telle sorte que sa profondeur augmente en direction de l'embouchure moins fortement qu'elle ne diminue ensuite à nouveau.

6. Endoprothèse selon l'une des revendications précédentes, caractérisée en ce qu'au moins l'élément en forme de tige (1, 2, 3, 4) est cintré d'une manière similaire à un os naturel de la cuisse.

7. Endoprothèse selon l'une des revendications précédentes, caractérisée en ce que le perçage (9) et l'embout (8) forment un système d'ajustement forcé.

8. Endoprothèse selon l'une des revendications précédentes, caractérisée en ce que les éléments de tige (1, 2, 3, 4) possèdent chacun un canal longitudinal servant à loger un tirant pour serrer les éléments de tige.

9. Endoprothèse selon l'une des revendications précédentes, caractérisée en ce qu'un élément de tige (1) possède une tête d'articulation artificielle (5).

10. Endoprothèse selon l'une des revendications précédentes, caractérisée en ce que sur un élément de tige (4) est formé un clou à os (7), qui sert à ancrer l'élément de tige (4) dans un os naturel.
